# EUROPEAN PATENT APPLICATION

(11) **EP 4 663 747 A1**
(43) Date of publication of application: **17.12.2025**
(21) Application number: 24753272.4
(22) Date of filing: 02.02.2024
(51) Int. Cl.: C12N 1/21, C12P 7/62, C12N 15/31

(54) **TRANSFORMED MICROORGANISM, AND METHOD FOR PRODUCING POLYHYDROXYALKANOIC ACID**

(30) Priority: 06.02.2023 JP 2023015848
(71) Applicant: Kaneka Corporation, Osaka-shi, Osaka 530-8288 (JP)
(72) Inventor: KATO, Saya, Hyogo 676-8688 (JP); ARIKAWA, Hisashi, Hyogo 676-8688 (JP); SATO, Shunsuke, Hyogo 676-8688 (JP)
(74) Representative: Vossius & Partner Patentanwälte Rechtsanwälte mbB
(86) International application number: PCT/JP2024/003568
(87) International publication number: WO 2024/166829

(57) **Abstract**

A transformed microorganism having an ability to produce a polyhydroxyalkanoate includes a polyhydroxyalkanoate synthase gene and a gene encoding a chaperone belonging to the ClpB family. The gene encoding the chaperone belonging to the ClpB family is a gene which has been introduced into the transformed microorganism or whose expression has been enhanced in the transformed microorganism. The chaperone belonging to the ClpB family may be derived from the genus *Cupriavidus, Escherichia,* or *Saccharomyces.* A polyhydroxyalkanoate can be produced by culturing the transformed microorganism.

## Description

### Technical Field

The present invention relates to a transformed microorganism having an ability to produce a polyhydroxyalkanoate and a polyhydroxyalkanoate production method using the transformed microorganism.

### Background Art

In recent years, there has been an increasing awareness of environmental problems. Against this background, biodegradable plastics are attracting attention as materials with low environmental impact. Examples of biodegradable plastics include polyhydroxyalkanoates (hereinafter also referred to as PHAs) produced by microorganisms. Not only do PHAs are biodegradable, but they can be produced using sugars or oils as raw materials. Thus, PHAs are expected to be industrially used as non-petroleum materials. However, the cost of PHA production is still high, and there is a need to increase the PHA productivity and reduce the production cost.

Chaperones are known as proteins that facilitate the formation of proper conformational structures of proteins. If proteins are misfolded due to a stress such as heat or an oxidative stress, chaperones unfold the misfolded proteins and assist in the refolding of the proteins. Low-molecular-weight chaperones such as IbpA and IbpB copolymerize with and stabilize denatured proteins and thereby prevent aggregation of the proteins. Chaperone GroESL or DnaKJ included in Hsp60 or Hsp70 assists in protein refolding as well as preventing protein aggregation. Chaperone ClpB functions to unfold and disaggregate aggregated proteins.

Non-Patent Literature 1 reports that overexpression of chaperone GroESL in *Cupriavidus necator* resulted in an increase of 9 to 18% in isopropanol productivity.

### Citation List

### Non-Patent Literature

NPL 1: Metab. Eng., 42, 74-84 (2017)

### Summary of Invention

### Technical Problem

As mentioned above, overexpression of chaperone GroESL in a microorganism has been reported to increase the isopropanol productivity. However, there has been no report of the impact of chaperones on the polyhydroxyalkanoate productivity.

In view of the above circumstances, the present invention aims to provide a transformed microorganism with increased polyhydroxyalkanoate productivity and a method for producing a polyhydroxyalkanoate by culturing the microorganism.

### Solution to Problem

As a result of intensive studies with the goal of solving the above problem, the present inventors have found that the polyhydroxyalkanoate productivity of a microorganism having polyhydroxyalkanoate-producing ability can be increased by introducing a gene encoding a chaperone belonging to the ClpB family into the microorganism or enhancing the expression of the gene in the microorganism. Based on this finding, the inventors have completed the present invention.

Specifically, the present invention relates to a transformed microorganism having an ability to produce a polyhydroxyalkanoate, the transformed microorganism including:
a polyhydroxyalkanoate synthase gene; and
a gene encoding a chaperone belonging to the ClpB family, wherein
the gene encoding the chaperone belonging to the ClpB family is a gene which has been introduced into the transformed microorganism or whose expression has been enhanced in the transformed microorganism.

The present invention also relates to a polyhydroxyalkanoate production method for producing a polyhydroxyalkanoate, the polyhydroxyalkanoate production method including the step of culturing the transformed microorganism.

### Advantageous Effects of Invention

The present invention can provide a transformed microorganism with increased polyhydroxyalkanoate productivity and a method for producing a polyhydroxyalkanoate by culturing the microorganism.

### Description of Embodiments

Hereinafter, an embodiment of the present invention will be described. The present invention is not limited to the embodiment described below.

The present disclosure relates to a transformed microorganism having an ability to produce a polyhydroxyalkanoate (hereinafter also referred to as a PHA). The present disclosure further relates to a method for producing a PHA by culturing the transformed microorganism.

The transformed microorganism according to the present disclosure is a PHA-producing microorganism which has a PHA synthase gene and into which a gene encoding a chaperone belonging to the ClpB family has been introduced or in which the expression of the chaperone gene has been enhanced.

The host of the transformed microorganism according to the present disclosure may be a wild strain inherently possessing a PHA synthase gene, a mutant strain obtained by artificially mutating the wild strain, or a transformed strain having an exogenous PHA synthase gene introduced by a genetic engineering technique.

Examples of the host of the transformed microorganism according to the present disclosure include bacteria belonging to the genus *Ralstonia, Cupriavidus, Wautersia, Aeromonas, Escherichia, Alcaligenes,* or *Pseudomonas.* In terms of safety and PHA productivity, bacteria belonging to the genus *Ralstonia, Cupriavidus, Wautersia,* or *Escherichia* are preferred, and microorganisms belonging to the genus *Cupriavidus* are even more preferred. Particularly preferred is *Cupriavidus necator.*

### (PHA)

The PHA produced by the transformed microorganism according to the present disclosure is not limited to a particular type and may be any PHA that can be produced by microorganisms. Preferred examples of the PHA include: a homopolymer of one monomer selected from 3-hydroxyalkanoates having 4 to 16 carbon atoms; a copolymer of two or more monomers selected from 3-hydroxyalkanoates having 4 to 16 carbon atoms; a copolymer of one monomer selected from 3-hydroxyalkanoates having 4 to 16 carbon atoms and another hydroxyalkanoate (such as a 2-hydroxyalkanoate, 4-hydroxyalkanoate, 5-hydroxyalkanoate, or 6-hydroxyalkanoate having 4 to 16 carbon atoms); and a copolymer of two or more monomers selected from 3-hydroxyalkanoates having 4 to 16 carbon atoms and another hydroxyalkanoate.

A particularly preferred PHA is a homopolymer of a 3-hydroxyalkanoate having 4 carbon atoms or a copolymer containing a 3-hydroxyalkanoate having 4 carbon atoms. Examples of such a PHA include, but are not limited to, P(3HB) which is a homopolymer of 3-hydroxybutyrate (abbreviated as 3HB), P(3HB-co-3HV) which is a copolymer of 3HB and 3-hydroxyvalerate (abbreviated as 3HV), P(3HB-co-3HH) (abbreviated as P3HB3HH) which is a copolymer of 3HB and 3-hydroxyhexanoate (abbreviated as 3HH), P(3HB-co-4HB) which is a copolymer of 3HB and 4-hydroxybutyrate (abbreviated as 4HB), and a PHA containing lactic acid (abbreviated as LA) as a constituent (an example of this PHA is P(LA-co-3HB) which is a copolymer of 3HB and LA). Among these, P3HB3HH is preferred since this polymer has a wide range of applications.

The type of the PHA to be produced can be chosen as appropriate according to the intended purpose and can be changed depending on factors such as the type of the PHA synthase gene possessed by or introduced into the microorganism used, the type of the metabolic gene involved in the PHA synthesis, and the culture conditions.

### (PHA Synthase Gene)

The PHA synthase (PhaC) gene of the transformed microorganism according to the present disclosure may be a gene inherently possessed by the host or an exogenous gene introduced into the host. Examples of the PHA synthase gene include, but are not limited to: PHA synthase genes derived from *Aeromonas caviae, Aeromonas hydrophila, Pseudomonas* SP 61-3, or *Cupriavidus necator;* a chimeric PHA synthase gene combining two or more of these PHA synthase genes; and a gene encoding a protein having an amino acid sequence having a sequence identity of at least 90% with the amino acid sequence of any one of the PHA synthase genes.

The sequence identity is preferably 95% or more, more preferably 97% or more, and even more preferably 99% or more.

The transformed microorganism according to the present disclosure may have one PHA synthase gene or a plurality of PHA synthase genes. When the transformed microorganism has a plurality of PHA synthase genes, they may be the same or different genes.

### (Gene Encoding Chaperone Belonging to ClpB Family)

The transformed microorganism according to the present disclosure is a microorganism into which a gene encoding a chaperone belonging to the ClpB family has been introduced or in which the expression of the gene has been enhanced. The introduction of the gene can increase the PHA productivity of the transformed microorganism. The transformed microorganism can produce a PHA at high productivity even under high stress conditions such as relatively high culture temperature conditions.

GroESL and DnaKJ, which are typical chaperones, have the function of assisting in the refolding of proteins, while the chaperone used in the present disclosure, which belongs to the ClpB family, does not have the function of assisting in the refolding of proteins but has the function of unfolding aggregated proteins by means of ATP.

In the present disclosure, the "ClpB family" includes homologues of ClpB. Examples of such homologues include HSP104 possessed by yeasts.

Examples of the gene encoding the chaperone belonging to the ClpB family include, but are not limited to, a gene encoding ClpB derived from the genus *Cupriavidus* (in particular, *Cupriavidus necator*)*,* a gene encoding ClpB derived from the genus *Escherichia* (in particular, *Escherichia coli*)*,* and a gene encoding HSP104 derived from the genus *Saccharomyces* (in particular, *Saccharomyces cerevisiae*)*.*

More specifically, the *Cupriavidus necator-derived* ClpB is preferably ClpB having the amino acid sequence of SEQ ID NO: 1 or a protein that has an amino acid sequence having a sequence identity of at least 65% with the amino acid sequence of SEQ ID NO: 1 and that has ClpB activity. The *Escherichia coli*-derived ClpB is preferably ClpB having the amino acid sequence of SEQ ID NO: 2 or a protein that has an amino acid sequence having a sequence identity of at least 65% with the amino acid sequence of SEQ ID NO: 2 and that has ClpB activity. The sequence identity between the amino acid sequence of SEQ ID NO: 1 and the amino acid sequence of SEQ ID NO: 2 is 67%.

In addition, the *Saccharomyces cerevisiae*-derived HSP104 is preferably HSP104 having the amino acid sequence of SEQ ID NO: 3 or a protein that has an amino acid sequence having a sequence identity of at least 90% with the amino acid sequence of SEQ ID NO: 3 and that has HSP104 activity.

The sequence identity is preferably 70% or more, more preferably 80% or more, even more preferably 90% or more, still even more preferably 95% or more, particularly preferably 97% or more, and most preferably 99% or more.

### (Gene Introduction)

Introduction of the target gene into the host is not limited to using a particular method. Methods that can be employed include: a method in which the target gene is directly inserted onto a chromosome of the host or a gene on the chromosome is replaced by the target gene; a method in which the target gene is directly inserted onto a megaplasmid possessed by the host or a gene on the megaplasmid is replaced by the target gene; and a method in which the target gene is attached to a vector such as a plasmid, phage, or phagemid and the vector with the gene is introduced into the host. Two or more of these methods may be used in combination.

In view of the stability of the introduced gene, it is preferable to use the method in which the target gene is directly inserted onto a chromosome or a megaplasmid of the host or a gene on the chromosome or the megaplasmid is replaced by the target gene, and it is more preferable to use the method in which the target gene is directly inserted onto a chromosome of the host or a gene on the chromosome is replaced by the target gene.

For reliable expression of the introduced gene, it is preferable to introduce the target gene in such a manner that the target gene is located downstream of a "gene expression regulatory sequence" inherently possessed by the host or downstream of an exogenous "gene expression regulatory sequence". The term "gene expression regulatory sequence" as used herein refers to a DNA sequence containing a base sequence that controls the level of transcription of the gene (an example of this base sequence is a promoter sequence) and/or a base sequence that regulates the level of translation of messenger RNA transcribed from the gene (an example of this base sequence is a Shine-Dalgarno sequence). The "gene expression regulatory sequence" used may be any suitable naturally-occurring base sequence or an artificially constructed or altered base sequence.

Examples of the promoter sequence or Shine-Dalgarno sequence contained in the "gene expression regulatory sequence" include, but are not limited to, the base sequences of SEQ ID NOS: 10 to 15 and base sequences containing a part of any of the base sequences of SEQ ID NOS: 10 to 15.

Replacement, deletion, insertion, and/or addition made to at least a part of the genome DNA can be accomplished by a method known to those skilled in the art. Typical examples of the method include a method using a transposon and the mechanism of homologous recombination (Ohman et al., J. Bacteriol., 162: 1068-1074 (1985) and a method based on site-specific integration caused by the mechanism of homologous recombination and on loss due to second homologous recombination (Noti et al., Methods Enzymol., 154: 197-217 (1987)). A method may also be used in which the sacB gene derived from *Bacillus subtilis* is allowed to coexist and thus in which a microbial strain having lost a gene due to second homologous recombination is easily isolated as a sucrose-resistant strain (Schweizer, Mol. Microbiol., 6: 1195-1204 (1992), Lenz et al., J. Bacteriol., 176: 4385-4393 (1994)). Another alternative method is genome editing using a CRISPR/Cas9 system for altering the target DNA (Y. Wang et al., ACS Synth Biol., 2016, 5(7): 721-732). In the CRISPR/Cas9 system, the guide RNA (gRNA) has a sequence capable of binding to a part of the base sequence of the genome DNA to be altered, and serves to carry the Cas9 to the target.

Introduction of a vector into cells is not limited to using a particular method. Examples of vector introduction methods include calcium chloride transformation, electroporation, polyethylene glycol transformation, and spheroplast transformation.

### (Enhancement of Gene Expression)

Enhancement of the expression of the gene encoding the chaperone belonging to the ClpB family is not limited to using a particular method. The level of expression can be increased by inserting a gene expression regulatory sequence upstream of the chaperone-encoding gene endogenously present on the chromosome or introducing a copy of the endogenous chaperone-encoding gene into a site different from that of the original endogenous chaperone-encoding gene. Alternatively, a mutation may be introduced into the endogenous chaperone-encoding gene to increase the ClpB activity provided by the gene. The above methods may be used in combination.

In the case where a gene expression regulatory sequence is inserted upstream of the endogenous chaperone-encoding gene on the chromosome, the insertion can be accomplished using a known method. For example, homologous recombination can be used. The gene expression regulatory sequence used may be a gene expression regulatory sequence as previously described.

In the case where a copy of the endogenous chaperone-encoding gene is introduced into a site different from that of the original endogenous chaperone-encoding gene, the introduction is not limited to using a particular method. Any of the following methods may be employed: a method in which the copy is directly inserted onto the chromosome of the host or a gene on the chromosome is replaced by the copy; a method in which the copy is introduced onto a megaplasmid possessed by the host; and a method in which the copy is attached to a vector such as a plasmid, phage, or phagemid and the vector with the copy is introduced into the host. Two or more of these methods may be used in combination. Since the introduced plasmid could be lost during culture, it is preferable that the copy of the endogenous chaperone-encoding gene be inserted onto the chromosome of the host or that a gene on the chromosome be replaced by the copy. The introduction, insertion, replacement, or attachment described above can be accomplished using a known method. For example, homologous recombination can be used to replace a gene on the chromosome of the host by the copy of the chaperone-encoding gene or insert the copy onto the chromosome of the host.

It is preferable that a gene expression regulatory sequence involved in the expression of the introduced copy of the endogenous chaperone-encoding gene be located upstream of the copy. The gene expression regulatory sequence joined to and located upstream of the endogenous chaperone-encoding gene may be a gene expression regulatory sequence inherently possessed by the host, any suitable naturally-occurring gene expression regulatory sequence, or an artificially constructed or altered gene expression regulatory sequence.

The gene expression regulatory sequence used for the endogenous chaperone-encoding gene is not limited to a particular type. A gene expression regulatory sequence located upstream of the endogenous chaperone-encoding gene may be introduced together with the gene, or a suitable gene expression regulatory sequence may be selected, joined to the gene, and introduced into the host. When the copy of the endogenous chaperone-encoding gene is inserted onto the chromosome of the host, the insertion may be done in such a way that the gene is joined to a gene expression regulatory sequence inherently present on the chromosome of the host. The gene expression regulatory sequence to be selected can be a gene expression regulatory sequence as previously described.

In the case where a mutation is introduced into the endogenous chaperone-encoding gene, the mutation introduction can be accomplished using a known method. For example, a mutation-introduced gene can be obtained by error-prone PCR using the chaperone-encoding gene as a template or by PCR using the chaperone-encoding gene as a template together with a primer into which a mutation has been introduced.

### (Thermophile-Derived phaA Gene and phaB Gene)

The transformed microorganism according to the present disclosure preferably has a phaA gene and a phaB gene. The phaA gene and/or phaB gene may be a phaA gene inherently possessed by the host and/or a phaB gene inherently possessed by the host. Alternatively, a gene encoding PhaA derived from a thermophile and/or a gene encoding PhaB derived from a thermophile may be introduced into the host.

The phaA gene is a gene encoding β-ketothiolase (PhaA) and the phaB gene is a gene encoding acetoacetyl CoA reductase (PhaB). These genes are possessed by many of the microorganisms that can inherently accumulate PHAs, and are involved in biosynthesis of 3HB-CoA which is the most typical substrate for PHA biosynthesis. Specifically, PhaA is involved in catalysis of a reaction in which two molecules of acetyl CoA are condensed to form acetoacetyl CoA, and PhaB is involved in catalysis of a reaction in which acetoacetyl CoA is reduced to form 3HB-CoA.

Examples of the gene encoding thermophile-derived β-ketothiolase (PhaA) include, but are not limited to: a gene encoding PhaA derived from *Cupriavidus* sp. strain S-6 and having the amino acid sequence of SEQ ID NO: 4; a gene encoding PhaA derived from *Caldimonas manganoxidans* and having the amino acid sequence of SEQ ID NO: 5; a gene encoding PhaA derived from *Schlegelella thermodepolymerans* and having the amino acid sequence of SEQ ID NO: 6; and a gene having a base sequence encoding a protein that has an amino acid sequence having a sequence identity of at least 90% with the amino acid sequence of any one of SEQ ID NOS: 4 to 6 and that exhibits β-ketothiolase activity.

The sequence identity with the amino acid sequence of SEQ ID NO: 4 is preferably 90% or more, more preferably 95% or more, even more preferably 97% or more, and particularly preferably 99% or more. The sequence identity with the amino acid sequence of SEQ ID NO: 5 or 6 is also preferably 90% or more, more preferably 95% or more, even more preferably 97% or more, and particularly preferably 99% or more.

Examples of the gene encoding thermophile-derived acetoacetyl CoA reductase (PhaB) include, but are not limited to: a gene encoding PhaB derived from *Cupriavidus* sp. strain S-6 and having the amino acid sequence of SEQ ID NO: 7; a gene encoding PhaB derived from *Caldimonas manganoxidans* and having the amino acid sequence of SEQ ID NO: 8; a gene encoding PhaB derived from *Schlegelella thermodepolymerans* and having the amino acid sequence of SEQ ID NO: 9; and a gene having a base sequence encoding a protein that has an amino acid sequence having a sequence identity of at least 90% with the amino acid sequence of any one of SEQ ID NOS: 7 to 9 and that exhibits acetoacetyl CoA reductase activity.

The sequence identity with the amino acid sequence of SEQ ID NO: 7 is preferably 90% or more, more preferably 95% or more, even more preferably 97% or more, still even more preferably 98% or more, and particularly preferably 99% or more. The sequence identity with the amino acid sequence of SEQ ID NO: 8 or 9 is also preferably 90% or more, more preferably 95% or more, even more preferably 97% or more, and particularly preferably 99% or more.

The introduction of the PhaA-encoding gene and/or PhaB-encoding gene can be accomplished by any of the gene introduction methods as previously described.

### (Production of PHA)

Culturing the transformed microorganism according to the present disclosure allows the microbial cells to accumulate a PHA. The culture of the transformed microorganism according to the present disclosure can be conducted by an ordinary microbial culture method, and it is sufficient that the transformed microorganism be cultured in a culture medium containing a suitable carbon source. There are no particular limitations on the composition of the culture medium, the way of adding the carbon source, the scale of the culture, the conditions of aeration and stirring, the culture temperature, the culture time, etc. It is preferable to add the carbon source to the culture medium continuously or intermittently.

The transformed microorganism according to the present disclosure can produce a PHA at high productivity even under relatively high culture temperature conditions. For example, the transformed microorganism can achieve high productivity even when cultured at 35°C or above.

The carbon source used for the culture may be any carbon source that can be assimilated by the transformed microorganism according to the present disclosure. Examples of the carbon source include, but are not limited to: sugars such as glucose, fructose, and sucrose; oils such as palm and palm kernel oils (including palm olein, palm double olein, and palm kernel olein which are low-melting-point fractions obtained through fractionation of palm oil and palm kernel oil), corn oil, coconut oil, olive oil, soybean oil, rapeseed oil, and Jatropha oil; fractions of these oils; by-products formed during refining of these oils; fatty acids such as lauric acid, oleic acid, stearic acid, palmitic acid, and myristic acid; derivatives of these fatty acids; and glycerol. When an oil as mentioned above is used, part or all of the oil may be a degraded oil. The term "degraded oil" refers to an oil that has been thermally denatured or that has been altered by a reaction with oxygen and/or water under heating. The term "degraded oil" is not limited to a particular type of oil but covers various oils called waste oil, discarded oil, waste edible oil, discarded edible oil, waste vegetable oil, or used oil. In the case where the transformed microorganism according to the present disclosure can assimilate gases such as carbon dioxide, carbon monoxide, and methane or alcohols such as methanol and ethanol, any of these gases or alcohols can be used as the carbon source.

In the PHA production in the present disclosure, the microorganism is preferably cultured using a culture medium containing the carbon source as described above and other nutrient sources including a nitrogen source, an inorganic salt, and another organic nutrient source. Examples of the nitrogen source include, but are not limited to: ammonia; ammonium salts such as ammonium chloride, ammonium sulfate, and ammonium phosphate; peptone; meat extracts; and yeast extracts. Examples of the inorganic salt include potassium dihydrogen phosphate, disodium hydrogen phosphate, magnesium phosphate, magnesium sulfate, and sodium chloride. Examples of the other organic nutrient source include: amino acids such as glycine, alanine, serine, threonine, and proline; and vitamins such as vitamin B1, vitamin B12, and vitamin C.

After the transformed microorganism is cultured for an adequate time to allow the microbial cells to accumulate a PHA, the PHA can be collected from the microbial cells using a known method. The collection method is not limited to using a particular technique. For example, after the end of the culture, the microbial cells are separated from the culture fluid by means such as a centrifuge or a separation membrane, and the separated microbial cells are dried. Subsequently, the PHA is extracted from the dried cells by means of an organic solvent such as chloroform, and cellular components are removed from the PHA-containing organic solvent solution by a process such as filtration. A poor solvent such as methanol or hexane is added to the filtrate to precipitate the PHA, then the supernatant is removed by filtration or centrifugation, and the PHA is dried and collected. Alternatively, the PHA may be collected by dissolving cellular components other than the PHA in water with the aid of a surfactant, an alkali, or an enzyme, then separating the PHA particles from the aqueous phase by filtration or centrifugation, and drying the separated PHA particles.

In the following items, preferred aspects of the present disclosure are listed. The present invention is not limited to the following items.

### [Item 1]

A transformed microorganism having an ability to produce a polyhydroxyalkanoate, the transformed microorganism including:
a polyhydroxyalkanoate synthase gene; and
a gene encoding a chaperone belonging to the ClpB family, wherein
the gene encoding the chaperone belonging to the ClpB family is a gene which has been introduced into the transformed microorganism or whose expression has been enhanced in the transformed microorganism.

### [Item 2]

The transformed microorganism according to item 1, wherein the chaperone belonging to the ClpB family has an amino acid sequence having a sequence identity of 65 to 100% with an amino acid sequence of SEQ ID NO: 1 or 2.

### [Item 3]

The transformed microorganism according to item 1 or 2, wherein the chaperone belonging to the ClpB family is derived from the genus *Cupriavidus* or *Escherichia.*

### [Item 4]

The transformed microorganism according to item 3, wherein the chaperone belonging to the ClpB family is derived from *Cupriavidus necator* or *Escherichia coli.*

### [Item 5]

The transformed microorganism according to item 1 or 2, wherein the transformed microorganism belongs to the genus *Cupriavidus.*

### [Item 6]

The transformed microorganism according to any one of items 1 to 5, including an introduced gene encoding PhaA derived from a thermophile and/or an introduced gene encoding PhaB derived from a thermophile.

### [Item 7]

The transformed microorganism according to item 6, wherein the thermophile is *Cupriavidus* sp. strain S-6.

### [Item 8]

A polyhydroxyalkanoate production method for producing a polyhydroxyalkanoate, the polyhydroxyalkanoate production method including the step of culturing the transformed microorganism according to any one of items 1 to 7.

### [Item 9]

The polyhydroxyalkanoate production method according to item 8, wherein the polyhydroxyalkanoate is a copolymer of two or more hydroxyalkanoates.

### [Item 10]

The polyhydroxyalkanoate production method according to item 9, wherein the polyhydroxyalkanoate is a copolymer containing 3-hydroxyhexanoate as a monomer unit.

### Examples

Hereinafter, the present invention will be described in more detail using examples. The present invention is not limited to the examples.

The overall genetic manipulation can be carried out, for example, in a manner as taught in Molecular Cloning (Cold Spring Harbor Laboratory Press (1989)). The enzymes, cloning hosts, and other materials used in the genetic manipulation can be purchased from market suppliers and used according to the instructions given by the suppliers. The enzymes are not limited to particular types and may be any enzymes that can be used for genetic manipulation.

The KNK005/dZ/trc-J4b as used below is a strain in which: the phaC1 gene (PHA synthase gene), phaZ1 gene, phaZ2 gene, and phaZ6 gene on the chromosome of *Cupriavidus necator* H16 have been deleted; the expression of the (R)-specific enoyl-CoA hydratase gene (phaJ4b gene) on the chromosome has been enhanced; and a gene encoding a PHA synthase mutant (N149S/D171G mutant (NSDG)) derived from the genus *Aeromonas* and having the amino acid sequence of SEQ ID NO: 19 has been introduced. This strain can be prepared according to a method described in WO 2015/115619 A1.

### (Microorganism Preparation Example 1)

First, a plasmid for chaperone (ClpB) gene expression was prepared. The preparation was done as follows.

PCR using synthetic oligo DNA was carried out to obtain a DNA fragment (SEQ ID NO: 17) having a lacN17 promoter which is an altered lac promoter of *Escherichia coli.* The DNA fragment was digested with restriction enzymes EcoRI and MunI, and the resulting DNA fragment was joined to a plasmid vector pCUP2 which is described in WO 2007/049716 A1 and which was cleaved with MunI. A conjugate was selected in which the DNA fragment was joined to the pCUP2 in such an orientation that the restriction enzyme SpeI-recognition sequence of the pCUP2 was located downstream of the lacN17 promoter. In this manner, pCUP2-lacN17 was obtained.

Next, PCR using synthetic oligo DNA was carried out to obtain a DNA fragment (SEQ ID NO: 18) having a base sequence of a gene encoding ClpB having the amino acid sequence of SEQ ID NO: 1. The DNA fragment was digested with restriction enzymes MunI and SpeI, and the resulting DNA fragment was joined to the pCUP2-lacN17 cleaved with MunI and SpeI. In this manner, a plasmid pCUP2-lacN17-ClpBre for ClpB gene expression was obtained.

Subsequently, a ClpB expression-enhanced strain was prepared using the plasmid pCUP2-lacN17-ClpBre for ClpB gene expression. The preparation was done as described below. The strain obtained by introducing the plasmid pCUP2-lacN17-ClpBre for ClpB gene expression into the KNK005/dZ/trc-J4b was named "KNK005/dZ/trc-J4b/pCUP2-lacN17-ClpBre" (hereinafter also referred to as "PHA-producing microbial strain (1)").

The introduction of the plasmid vector into the cells was carried out by electroporation as described below. The gene introduction device used was Gene Pulser manufactured by Bio-Rad Laboratories, Inc., and the cuvette used was a 0.2-cm-gap cuvette also manufactured by Bio-Rad Laboratories, Inc. The cuvette was charged with 400 µl of competent cells and 20 µl of the expression vector and set on the pulse device, by which electric pulse was applied to the contents of the cuvette at a capacitance of 25 µF, a voltage of 1.5 kV, and a resistance value of 800 Ω. After the pulse application, the cell-containing fluid in the cuvette was subjected to shake culture in Nutrient Broth (manufactured by Difco Laboratories, Inc.) at 30°C for 3 hours and then to culture on a selection plate (Nutrient Agar manufactured by Difco Laboratories, Inc. and containing 100 mg/L kanamycin) at 30°C for 2 days. The PHA-producing microbial strain (1) grown on the selection plate was collected.

The PHA-producing microbial strain (1) is a strain in which: the phaC1 gene (PHA synthase gene), phaZ1 gene, phaZ2 gene, and phaZ6 gene on the chromosome of *Cupriavidus necator* H16 have been deleted; the expression of the (R)-specific enoyl-CoA hydratase gene on the chromosome has been enhanced; a gene encoding a PHA synthase mutant derived from the genus *Aeromonas* and having the amino acid sequence of SEQ ID NO: 19 has been introduced; and the expression of a gene encoding ClpB derived from the genus *Cupriavidus* and having the amino acid sequence of SEQ ID NO: 1 has been enhanced.

### (Microorganism Preparation Example 2)

First, a plasmid for chaperone (ClpB) gene expression was prepared. The preparation was done as follows. PCR using synthetic oligo DNA was carried out to obtain a DNA fragment (SEQ ID NO: 20) having a base sequence of a gene encoding ClpB having the amino acid sequence of SEQ ID NO: 2. The DNA fragment was digested with restriction enzymes MunI and SpeI, and the resulting DNA fragment was joined to pCUP2-lacN17 cleaved with MunI and SpeI. In this manner, a plasmid pCUP2-lacN17-ClpBec for ClpB gene expression was obtained.

Next, a ClpBec-introduced strain was prepared using the plasmid pCUP2-lacN17-ClpBec for ClpB gene expression. The preparation was done as follows: the plasmid pCUP2-lacN17-ClpBec for ClpB gene expression was introduced into the KNK005/dZ/trc-J4b by electroporation as described above. The strain thus obtained was named "KNK005/dZ/trc-J4b/pCUP2-lacN17-ClpBec" (hereinafter also referred to as "PHA-producing microbial strain (2)").

The PHA-producing microbial strain (2) is a strain in which: the phaC1 gene (PHA synthase gene), phaZ1 gene, phaZ2 gene, and phaZ6 gene on the chromosome of *Cupriavidus necator* H16 have been deleted; the expression of the (R)-specific enoyl-CoA hydratase gene on the chromosome has been enhanced; and a gene encoding a PHA synthase mutant derived from the genus *Aeromonas* and having the amino acid sequence of SEQ ID NO: 19 and a gene encoding ClpB derived from the genus *Escherichia* and having the amino acid sequence of SEQ ID NO: 2 have been introduced.

### (Microorganism Preparation Example 3)

First, a plasmid for chaperone (GroESL) gene expression was prepared. The preparation was done as follows. PCR using synthetic oligo DNA was carried out to obtain a DNA fragment (SEQ ID NO: 23) having a base sequence of a gene encoding GroES having the amino acid sequence of SEQ ID NO: 21 and GroEL having the amino acid sequence of SEQ ID NO: 22. The DNA fragment was digested with restriction enzymes MunI and SpeI, and the resulting DNA fragment was joined to pCUP2-lacN17 cleaved with MunI and SpeI. In this manner, a plasmid pCUP2-lacN17-GroESL for GroESL gene expression was obtained.

Next, a GroESL expression-enhanced strain was prepared using the plasmid pCUP2-lacN17-GroESL for GroESL gene expression. The preparation was done as follows: the plasmid pCUP2-lacN17-GroESL for GroESL gene expression was introduced into the KNK005/dZ/trc-J4b by electroporation as described above. The strain thus obtained was named "KNK005/dZ/trc-J4b/pCUP2-lacN17-GroESL" (hereinafter also referred to as "PHA-producing microbial strain (3)").

The PHA-producing microbial strain (3) is a strain in which: the phaC1 gene (PHA synthase gene), phaZ1 gene, phaZ2 gene, and phaZ6 gene on the chromosome of *Cupriavidus necator* H16 have been deleted; the expression of the (R)-specific enoyl-CoA hydratase gene on the chromosome has been enhanced; and a gene encoding a PHA synthase mutant derived from the genus *Aeromonas* and having the amino acid sequence of SEQ ID NO: 19 and a gene encoding GroESL derived from the genus *Cupriavidus* and having the amino acid sequences of SEQ ID NOS: 21 and 22 have been introduced.

### (Microorganism Preparation Example 4)

First, a plasmid for chaperone (DnaKJ) gene expression was prepared. The preparation was done as follows. PCR using synthetic oligo DNA was carried out to obtain a DNA fragment (SEQ ID NO: 26) having a base sequence of a gene encoding DnaK having the amino acid sequence of SEQ ID NO: 24 and DnaJ having the amino acid sequence of SEQ ID NO: 25. The DNA fragment was digested with restriction enzymes MunI and SpeI, and the resulting DNA fragment was joined to pCUP2-lacN17 cleaved with MunI and SpeI. In this manner, a plasmid pCUP2-lacN17-DnaKJ for DnaKJ gene expression was obtained.

Next, a DnaKJ-introduced strain was prepared using the plasmid pCUP2-lacN17-DnaKJ for DnaKJ gene expression. The preparation was done as follows: the plasmid pCUP2-lacN17-DnaKJ for DnaKJ gene expression was introduced into the KNK005/dZ/trc-J4b by electroporation as described above. The strain thus obtained was named "KNK005/dZ/trc-J4b/pCUP2-lacN17-DnaKJ" (hereinafter also referred to as "PHA-producing microbial strain (4)").

The PHA-producing microbial strain (4) is a strain in which: the phaC1 gene (PHA synthase gene), phaZ1 gene, phaZ2 gene, and phaZ6 gene on the chromosome of *Cupriavidus necator* H16 have been deleted; the expression of the (*R*)-specific enoyl-CoA hydratase gene on the chromosome has been enhanced; and a gene encoding a PHA synthase mutant derived from the genus *Aeromonas* and having the amino acid sequence of SEQ ID NO: 19 and a gene encoding DnaKJ derived from the genus *Escherichia* and having the amino acid sequences of SEQ ID NOS: 24 and 25 have been introduced.

### (Microorganism Preparation Example 5)

First, a plasmid for disruption of phaA and phaB genes was prepared. The preparation was done as follows.

PCR using synthetic oligo DNA was carried out to obtain a DNA fragment (SEQ ID NO: 27) having base sequences upstream and downstream of the phaA and phaB structural genes of *Cupriavidus necator* H16. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+phaABUD for phaAB gene disruption was prepared.

Next, a phaAB gene-disrupted strain was prepared using the plasmid vector pNS2X-sacB+phaABUD for phaAB gene disruption. The preparation was done as follows.

*Escherichia coli* S17-1 (ATCC 47055) was transformed with the plasmid vector pNS2X-sacB+phaABUD for phaAB gene disruption, and the resulting transformed microorganism was co-cultured with the KNK005/dZ/trc-J4b on Nutrient Agar (manufactured by Difco Laboratories, Inc.) to effect conjugal transfer.

The resulting culture fluid was inoculated into a Simmons agar medium (2 g/L sodium citrate, 5 g/L sodium chloride, 0.2 g/L magnesium sulfate heptahydrate, 1 g/L ammonium dihydrogen phosphate, 1 g/L dipotassium hydrogen phosphate, 15 g/L agar, pH = 6.8) containing 250 mg/L kanamycin, and strains grown on the agar medium were selectively collected. Thus, a strain having the plasmid integrated into the chromosome of the KNK005/dZ/trc-J4b was obtained. This strain was cultured in Nutrient Broth (manufactured by Difco Laboratories, Inc.) for two generations, after which the culture broth was diluted and applied onto Nutrient Agar containing 15% sucrose. Strains grown on the Nutrient Agar were obtained as strains from which the plasmid was lost. PCR and analysis using a DNA sequencer were further carried out to isolate one strain in which the phaAB gene on the chromosome was deleted. This gene-disrupted strain was named "KNK005/dZ/trc-J4b/dphaAB".

A plasmid for introduction of phaA and phaB genes was also prepared. The preparation was done as follows.

PCR using synthetic oligo DNA was carried out to obtain a DNA fragment (SEQ ID NO: 28) having base sequences upstream and downstream of the phaA and phaB structural genes of *Cupriavidus necator* H16, a base sequence of a gene encoding PhaA having the amino acid sequence of SEQ ID NO: 4, and a base sequence of a gene encoding phaB having the amino acid sequence of SEQ ID NO: 7. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+phaAU-phaABsp-phaBD for introduction of phaA and phaB genes was prepared.

Next, the plasmid vector pNS2X-sacB+phaAU-phaABsp-phaBD for introduction of phaA and phaB genes was introduced into the KNK005/dZ/trc-J4b/dphaAB by procedures using conjugal transfer as described above. The subsequent culture and selection on Nutrient Agar containing 15% sucrose were carried out as described above to isolate one strain into which were introduced the gene encoding PhaA having the amino acid sequence of SEQ ID NO: 4 and the gene encoding PhaB having the amino acid sequence of SEQ ID NO: 7. The strain thus obtained was named "KNK005/dZ/trc-J4b/dphaAB::phaABsp".

Furthermore, a ClpBre expression-enhanced strain was prepared using the plasmid pCUP2-lacN17-ClpBre for ClpB gene expression. The preparation was done as follows.

The plasmid pCUP2-lacN17-ClpBre for ClpB gene expression was introduced into the KNK005/dZ/trc-J4b/dphaAB::phaABsp by electroporation as described above. The strain thus obtained was named "KNK005/dZ/trc-J4b/dphaAB::phaABsp/pCUP2-lacN17-ClpBre" (hereinafter also referred to as "PHA-producing microbial strain (5)").

The PHA-producing microbial strain (5) is a strain in which: the phaC1 gene (PHA synthase gene), phaZ1 gene, phaZ2 gene, phaZ6 gene, and phaAB gene on the chromosome of *Cupriavidus necator* H16 have been deleted; the expression of the (*R*)-specific enoyl-CoA hydratase gene on the chromosome has been enhanced; a gene encoding a PHA synthase mutant derived from the genus *Aeromonas* and having the amino acid sequence of SEQ ID NO: 19, a gene encoding phaA derived from *Cupriavidus* sp. strain S-6 and having the amino acid sequence of SEQ ID NO: 4, and a gene encoding phaB derived from *Cupriavidus* sp. strain S-6 and having the amino acid sequence of SEQ ID NO: 7 have been introduced; and the expression of a gene encoding ClpB derived from the genus *Cupriavidus* and having the amino acid sequence of SEQ ID NO: 1 has been enhanced.

### (Microorganism Preparation Example 6)

Furthermore, a ClpBec-introduced strain was prepared using the plasmid pCUP2-lacN17-ClpBec for ClpB gene expression. The preparation was done as follows.

The plasmid pCUP2-lacN17-ClpBre for ClpB gene expression was introduced into the KNK005/dZ/trc-J4b/dphaAB::phaABsp by electroporation as described above. The strain thus obtained was named "KNK005/dZ/trc-J4b/dphaAB::phaABsp/pCUP2-lacN17-ClpBec" (hereinafter also referred to as "PHA-producing microbial strain (6)").

The PHA-producing microbial strain (6) is a strain in which: the phaC1 gene (PHA synthase gene), phaZ1 gene, phaZ2 gene, phaZ6 gene, and phaAB gene on the chromosome of *Cupriavidus necator* H16 have been deleted; the expression of the (*R*)-specific enoyl-CoA hydratase gene on the chromosome has been enhanced; and a gene encoding a PHA synthase mutant derived from the genus *Aeromonas* and having the amino acid sequence of SEQ ID NO: 19, a gene encoding phaA derived from *Cupriavidus* sp. strain S-6 and having the amino acid sequence of SEQ ID NO: 4, a gene encoding phaB derived from *Cupriavidus* sp. strain S-6 and having the amino acid sequence of SEQ ID NO: 7, and a gene encoding ClpB derived from the genus *Escherichia* and having the amino acid sequence of SEQ ID NO: 2 have been introduced.

### (Microorganism Preparation Example 7)

Furthermore, a GroESL-introduced strain was prepared using the plasmid pCUP2-lacN17-GroESL for GroESL gene expression. The preparation was done as follows.

The plasmid pCUP2-lacN17-GroESL for GroESL gene expression was introduced into the KNK005/dZ/trc-J4b/dphaAB::phaABsp by electroporation as described above. The strain thus obtained was named "KNK005/dZ/trc-J4b/dphaAB::phaABsp/pCUP2-lacN17-GroESL" (hereinafter also referred to as "PHA-producing microbial strain (7)").

The PHA-producing microbial strain (7) is a strain in which: the phaC1 gene (PHA synthase gene), phaZ1 gene, phaZ2 gene, phaZ6 gene, and phaAB gene on the chromosome of *Cupriavidus necator* H16 have been deleted; the expression of the (*R*)-specific enoyl-CoA hydratase gene on the chromosome has been enhanced; and a gene encoding a PHA synthase mutant derived from the genus *Aeromonas* and having the amino acid sequence of SEQ ID NO: 19, a gene encoding phaA derived from *Cupriavidus* sp. strain S-6 and having the amino acid sequence of SEQ ID NO: 4, a gene encoding phaB derived from *Cupriavidus* sp. strain S-6 and having the amino acid sequence of SEQ ID NO: 7, and a gene encoding GroESL derived from the genus *Cupriavidus* and having the amino acid sequences of SEQ ID NOS: 21 and 22 have been introduced.

### (Microorganism Preparation Example 8)

Furthermore, a DnaKJ-introduced strain was prepared using the plasmid pCUP2-lacN17-DnaKJ for DnaKJ gene expression. The preparation was done as follows.

The plasmid pCUP2-lacN17-DnaKJ for DnaKJ gene expression was introduced into the KNK005/dZ/trc-J4b/dphaAB::phaABsp by electroporation as described above. The strain thus obtained was named "KNK005/dZ/trc-J4b/dphaAB::phaABsp/pCUP2-lacN17-DnaKJ" (hereinafter also referred to as "PHA-producing microbial strain (8)").

The PHA-producing microbial strain (8) is a strain in which: the phaC1 gene (PHA synthase gene), phaZ1 gene, phaZ2 gene, phaZ6 gene, and phaAB gene on the chromosome of *Cupriavidus necator* H16 have been deleted; the expression of the (*R*)-specific enoyl-CoA hydratase gene on the chromosome has been enhanced; and a gene encoding a PHA synthase mutant derived from the genus *Aeromonas* and having the amino acid sequence of SEQ ID NO: 19, a gene encoding phaA derived from *Cupriavidus* sp. strain S-6 and having the amino acid sequence of SEQ ID NO: 4, a gene encoding phaB derived from *Cupriavidus* sp. strain S-6 and having the amino acid sequence of SEQ ID NO: 7, and a gene encoding DnaKJ derived from the genus *Escherichia* and having the amino acid sequences of SEQ ID NOS: 24 and 25 have been introduced.

### (Example 1) PHA production by PHA-producing microbial strain (1)

Culture examination using the PHA-producing microbial strain (1) was conducted under the conditions described below.

### (Method for Measuring Amount of Accumulated PHA)

The percentage of accumulated PHA to dried microbial cells was measured as follows. The microbial cells were collected from the culture fluid by centrifugation. The collected microbial cells were washed with ethanol and freeze-dried to give dried microbial cells, the weight of which was measured. To 1 g of the dried microbial cells was added 100 ml of chloroform, and the microbial cells in chloroform were stirred at room temperature for a day to extract a PHA (PHA mixture) from the microbial cells. The residues of the microbial cells were removed by filtration, and the filtrate was concentrated using an evaporator to a total volume of 30 ml. To the concentrate was slowly added 90 ml of hexane, and the liquid mixture was left for 1 hour under gentle stirring. The PHA precipitated was collected by filtration and vacuum-dried at 50°C for 3 hours. The weight of the dried PHA was measured, and the amount of PHA production was calculated.

### (PHA Production Culture in Flask)

The seed culture medium was composed of 10 g/L meat extract, 10 g/L Bacto-Trypton, 2 g/L yeast extract, 9 g/L sodium dihydrogen phosphate dodecahydrate, 1.5 g/L dipotassium hydrogen phosphate, and 100 µg/L kanamycin sulfate.

The PHA production culture medium was composed of 11 g/L disodium hydrogen phosphate dodecahydrate, 1.9 g/L dipotassium hydrogen phosphate, 1.3 g/L ammonium sulfate, 5 mL/L magnesium solution, and 1 mL/L trace metal salt solution. The magnesium solution was prepared by dissolving 200 g/L magnesium sulfate heptahydrate in water. The trace metal salt solution was prepared by dissolving 0.218 g/L cobalt chloride hexahydrate, 16.2 g/L iron(III) chloride hexahydrate, 10.3 g/L calcium chloride dihydrate, 0.118 g/L nickel chloride hexahydrate, and 0.156 g/L copper sulfate pentahydrate in 0.1 N hydrochloric acid.

A volume of 50 µL of glycerol stock solution of the KNK005/dZ/trc-J4b/pCUP2-lacN17-ClpBre prepared in Microorganism Preparation Example 1 was inoculated into 10 mL of the seed culture medium, in which the strain was cultured under shaking at 30°C for 24 hours. The resulting culture fluid was used as a preculture fluid.

PHA production culture was conducted in a flask. A 500-mL shake flask was charged with 50 mL of the PHA production culture medium. Immediately before inoculation, 250 µL of the magnesium solution, 50 µL of the trace metal solution, and 1 g of palm kernel oil were added to the culture medium. After the preparation of the culture medium, 500 µL of the preculture fluid was inoculated into the shake flask, in which the strain was cultured under shaking at 36°C for 72 hours. After the end of the culture, the amount of PHA production was measured as described above. The amount of PHA production in Comparative Example 1 where the KNK005/dZ/trc-J4b was cultured under the same conditions is used as a reference value, and the amount of PHA production in Example 1 is shown as a relative value with respect to the reference value in Table 1.

### (Example 2) PHA production by PHA-producing microbial strain (2)

Culture examination using the PHA-producing microbial strain (2) was conducted under the same conditions as the culture examination of Example 1, and the amount of PHA production was measured as described above. As with Example 1, the amount of PHA production is shown as a relative value with respect to the reference value in Table 1.

### (Comparative Examples 1 to 3)

Culture examination using the KNK005/dZ/trc-J4b, the PHA-producing microbial strain (3), or the PHA-producing microbial strain (4) was conducted under the same conditions as the culture examination of Example 1, and the amount of PHA production was measured as described above. As with Example 1, the amount of PHA production is shown as a relative value with respect to the reference value in Table 1.

**[Table 1]**

| | Strain name | Phasin gene which was introduced or whose expression was enhanced | Relative value of amount of PHA production (%) |
|---|---|---|---|
| Ex. 1 | PHA-producing microbial strain (1) | ClpBre | 152 |
| Ex. 2 | PHA-producing microbial strain (2) | ClpBec | 156 |
| Comp. 1 | KNK005/dZ/trc-J4b | - | 100 |
| Comp. 2 | PHA-producing microbial strain (3) | GroESL | 106.0 |
| Comp. 3 | PHA-producing microbial strain (4) | DnaKJ | 91.3 |

Table 1 reveals the following findings. A comparison of Examples 1 and 2 with Comparative Example 1 shows that the amount of PHA production was greater in Examples 1 and 2 than in Comparative Example 1. This demonstrates that the introduction or enhanced expression of a gene encoding a chaperone belonging to the ClpB family increased the PHA productivity.

A comparison of Examples 1 and 2 with Comparative Examples 2 and 3 shows that the amount of PHA production was greater in Examples 1 and 2 than in Comparative Examples 2 and 3. This demonstrates that the introduction or enhanced expression of a gene encoding a chaperone belonging to the ClpB family has a greater effect on the increase in PHA productivity than the introduction or enhanced expression of a gene encoding another kind of chaperone such as GroESL or DnaKJ.

### (Example 3) PHA production by PHA-producing microbial strain (5)

Culture examination using the PHA-producing microbial strain (5) was conducted under the conditions described below.

### (PHA Production by High-Density Culture)

The seed culture medium was composed of 1 w/v% Meat-extract, 1 w/v% Bacto-Tryptone, 0.2 w/v% Yeast-extract, 0.9 w/v% Na₂HPO₄•12H₂O, and 0.15 w/v% KH₂PO₄ (pH = 6.8).

The preculture medium was composed of 1.1 w/v% Na₂HPO₄•12H₂O, 0.19 w/v% KH₂PO₄, 1.29 w/v% (NH₄)₂SO₄, 0.1 w/v% MgSO₄•7H₂O, 2.5 w/v% palm olein oil, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1 N hydrochloric acid).

The PHA production culture medium was composed of 0.385 w/v% Na₂HPO₄•12H₂O, 0.067 w/v% KH₂PO₄, 0.291 w/v% (NH₄)₂SO₄, 0.1 w/v% MgSO₄•7H₂O, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1 N hydrochloric acid).

PHA production culture was performed as follows. First, seed culture was carried out by inoculating a glycerol stock (50 µl) of the PHA-producing microbial strain (5) into the seed culture medium (10 ml) and culturing the strain for 24 hours. Next, the seed culture fluid was inoculated at a concentration of 1.0 v/v% into a 3-L jar fermenter (MDL-300, manufactured by B.E. Marubishi Co., Ltd.) charged with 1.8 L of the preculture medium. The fermenter was operated at a culture temperature of 30°C, a stirring speed of 500 rpm, and an aeration of 1.8 L/min, and the preculture was conducted for 28 hours during which the pH was controlled between 6.7 and 6.8. For the pH control, a 14% aqueous solution of ammonium hydroxide was used.

Next, the preculture fluid was inoculated at a concentration of 5.0 v/v% into a 5-L jar fermenter (MDS-U50, manufactured by B.E. Marubishi Co., Ltd.) charged with 2.5 L of the PHA production culture medium. The fermenter was operated at a culture temperature of 36°C, a stirring speed of 420 rpm, and an aeration of 2.1 L/min, and the pH was controlled between 6.7 and 6.8. For the pH control, a 25% aqueous solution of ammonium hydroxide was used. The carbon source was added intermittently. Palm olein oil was used as the carbon source. The culture was performed for 48 hours. The amount of PHA production was measured as described above. The amount of PHA production in Comparative Example 4 where the KNK005/dZ/trc-J4b was cultured under the same conditions is used as a reference value, and the amount of PHA production in Example 3 is shown as a relative value with respect to the reference value in Table 2.

### (Example 4) PHA production by PHA-producing microbial strain (6)

Culture examination using the PHA-producing microbial strain (6) was conducted under the same conditions as the culture examination of Example 3, and the amount of PHA production was measured as described above. As with Example 3, the amount of PHA production is shown as a relative value with respect to the reference value in Table 2.

### (Comparative Examples 4 to 7)

Culture examination using the KNK005/dZ/trc-J4b, the KNK005/dZ/trc-J4b/dphaAB::phaABsp, the PHA-producing microbial strain (7), or the PHA-producing microbial strain (8) was conducted under the same conditions as the culture examination of Example 1, and the amount of PHA production was measured as described above. As with Example 3, the amount of PHA production is shown as a relative value with respect to the reference value in Table 2.

**[Table 2]**

| | Strain name | phaAB gene | Phasin gene which was introduced or whose expression was enhanced | Relative value of amount of PHA production (%) |
|---|---|---|---|---|
| Ex. 3 | PHA-producing microbial strain (5) | phaABsp | ClpBre | 121 |
| Ex. 4 | PHA-producing microbial strain (6) | phaABsp | ClpBec | 122 |
| Comp. 4 | KNK005/dZ/trc-J4b | phaABre | - | 100 |
| Comp. 5 | KNK005/dZ/trc-J4b/dphaAB::phaABsp | phaABsp | - | 116 |
| Comp. 6 | PHA-producing microbial strain (7) | phaABsp | GroESL | 105 |
| Comp. 7 | PHA-producing microbial strain (8) | phaABsp | DnaKJ | 77 |

Table 2 reveals the following findings. A comparison of Examples 3 and 4 with Comparative Example 5 shows that the amount of PHA production was greater in Examples 3 and 4 than in Comparative Example 5. This demonstrates that the introduction or enhanced expression of a gene encoding a chaperone belonging to the ClpB family increased the PHA productivity.

A comparison of Examples 3 and 4 with Comparative Examples 6 and 7 shows that the PHA productivity was higher in Examples 3 and 4 than in Comparative Examples 6 and 7. This demonstrates that the introduction or enhanced expression of a gene encoding a chaperone belonging to the ClpB family has a greater effect on the increase in PHA productivity than the introduction or enhanced expression of a gene encoding another kind of chaperone such as GroESL or DnaKJ.

## Claims

1. A transformed microorganism having an ability to produce a polyhydroxyalkanoate, the transformed microorganism comprising:
a polyhydroxyalkanoate synthase gene; and
a gene encoding a chaperone belonging to the ClpB family, wherein
the gene encoding the chaperone belonging to the ClpB family is a gene which has been introduced into the transformed microorganism or whose expression has been enhanced in the transformed microorganism.

2. The transformed microorganism according to claim 1, wherein the chaperone belonging to the ClpB family has an amino acid sequence having a sequence identity of 65 to 100% with an amino acid sequence of SEQ ID NO: 1 or 2.

3. The transformed microorganism according to claim 1 or 2, wherein the chaperone belonging to the ClpB family is derived from the genus *Cupriavidus* or *Escherichia.*

4. The transformed microorganism according to claim 3, wherein the chaperone belonging to the ClpB family is derived from *Cupriavidus necator* or *Escherichia coli.*

5. The transformed microorganism according to claim 1 or 2, wherein the transformed microorganism belongs to the genus *Cupriavidus.*

6. The transformed microorganism according to claim 1 or 2, comprising an introduced gene encoding PhaA derived from a thermophile and/or an introduced gene encoding PhaB derived from a thermophile.

7. The transformed microorganism according to claim 6, wherein the thermophile is *Cupriavidus* sp. strain S-6.

8. A polyhydroxyalkanoate production method for producing a polyhydroxyalkanoate, the polyhydroxyalkanoate production method comprising the step of culturing the transformed microorganism according to claim 1 or 2.

9. The polyhydroxyalkanoate production method according to claim 8, wherein the polyhydroxyalkanoate is a copolymer of two or more hydroxyalkanoates.

10. The polyhydroxyalkanoate production method according to claim 9, wherein the polyhydroxyalkanoate is a copolymer containing 3-hydroxyhexanoate as a monomer unit.
